# EUROPÄISCHE PATENTSCHRIFT

(11) **EP 3 035 220 B1**
(45) Veröffentlichungstag und Bekanntmachung des Hinweises auf die Patenterteilung: **18.09.2024**
(21) Anmeldenummer: 14198598.6
(22) Anmeldetag: 17.12.2014
(51) Int. Cl.: G16H 30/20

(54) **Verfahren und System zur gemeinsamen Auswertung eines medizinischen Bilddatensatzes**
Method and system for joint evaluating a medicinal image database
Procédé et système pour l'analyse commune d'un jeu de données graphiques médicales

(43) Veröffentlichungstag der Anmeldung: 22.06.2016
(73) Patentinhaber: Siemens Healthineers AG, 91301 Forchheim (DE)
(72) Erfinder: Dominick, Lutz, 91330 Eggolsheim (DE); Ukis, Vladyslav, 90489 Nürnberg (DE)
(74) Vertreter: Siemens Healthineers Patent Attorneys

(56) Entgegenhaltungen:
- EP-A1- 2 735 982
- US-A- 5 187 790
- US-A1- 2006 235 936
- US-A1- 2011 107 270
- US-A1- 2011 126 127
- US-A1- 2011 282 686
- US-A1- 2013 208 955
- US-A1- 2014 282 624

## Beschreibung

Die Erfindung betrifft ein Verfahren nach dem Oberbegriff des des Anspruchs 1 zur gemeinsamen Auswertung eines medizinischen Bilddatensatzes an mindestens zwei Datenverarbeitungseinrichtungen, die über ein Datenübertragungsnetz verbunden sind. Die Erfindung bezieht sich weiterhin auf ein System nach dem Oberbegriff des Anspruchs 5 zur Durchführung des Verfahrens.

Ein solches Verfahren und eine solche Vorrichtung sind aus US 2011/126127 A1 bekannt. Aus US 5,187,790 A ist ferner ein multitasking- und multiuserfähiges Client-Server-Computersystem bekannt, bei dem ein Serverprozess vorübergehend die Charakteristik eines Clientprozesses imitiert, wenn ein Clientprozess einen Fernzugriff (Remote Procedure Call) auf den Serverprozess vornimmt.

In der Medizin kommen zunehmend arbeitsteilige Bearbeitungsabläufe zum Einsatz, bei denen digitale medizinische Bilddatensätze durch eine Mehrzahl von Benutzern (in der Regel medizinischen Experten aus dem gleichen Teilbereich oder unterschiedlichen Teilbereichen der Medizin) gemeinsam ausgewertet (befundet) werden. Diese Befundung erfolgt in der modernen Medizin regelmäßig elektronisch unter Nutzung von Datenverarbeitungseinrichtung (Computern).

Im Sinne einer hohen Arbeitseffizienz und eines hochqualifizierten Befundungsergebnisses ist es in der Regel sinnvoll, dass die Auswertung gleichzeitig durch alle beteiligten Benutzer stattfindet, insbesondere in einer Besprechungsumgebung, in der die Benutzer unmittelbar und in Echtzeit Informationen austauschen können. Typischerweise versammeln sich die beteiligten Experten zur gemeinsamen Auswertung eines Bilddatensatzes daher vor einem Bildschirm. Häufig ist es aber nicht möglich oder zumindest unrentabel, die beteiligten Benutzer zur gleichen Zeit an einem Ort zu versammeln. Vielmehr ist es oft wünschenswert, die gemeinsame Auswertung von medizinischen Bilddatensätzen räumlich entfernt unter Zuhilfenahme mehrerer Datenverarbeitungseinrichtungen vorzunehmen, die über ein Datenübertragungsnetz verbunden sind.

Erfahrungsgemäß ist es für die Effizienz eines solchen arbeitsteiligen Auswertungsprozesses wesentlich, dass für alle beteiligten Experten ohne wesentlichen Zeitverlust die gleiche Information verfügbar gemacht wird. Insbesondere ist essentiell, dass alle Beteiligten die gleiche Bildinformation sehen. Diese Anforderung ist durch heutige Datenverarbeitungssysteme und -verfahren regelmäßig nicht in zufriedenstellender Weise realisierbar.

Zwar ist nach heutiger Technik möglich, dass mehrere Benutzer gleichzeitig denselben Datensatz an ihren jeweils lokalen Datenverarbeitungseinrichtungen unter Nutzung jeweils lokaler Applikationen unabhängig voneinander zur selben Zeit betrachten und befunden können. Allerdings werden die jeweils von den einzelnen Bearbeitern erzeugten Befunde dann nicht oder nur mit erheblicher Zeitverzögerung für die jeweils anderen Benutzer sichtbar, so dass ein effektiver Informationsaustausch nicht möglich ist. Zudem besteht bei unabhängiger Bearbeitung des gleichen Bilddatensatzes durch mehrere Benutzer ein hohes Risiko, dass Arbeitsergebnisse eines Benutzers durch Aktionen eines anderen Benutzers überschrieben werden und somit verloren gehen.

Des Weiteren ist grundsätzlich möglich, dass einer der Benutzer seinen Bildschirminhalt (also die auf seinem Bildschirm angezeigte Information) einer anderen Datenverarbeitungseinrichtung unter Nutzung einer Remote-Screen-Technologie zur Verfügung stellt. Die Verteilung der Bildschirmanzeige über eine Remote-Screen-Technologie ist aber in der Regel mit einem erheblichen Qualitätsverlust der anzuzeigenden Bilddaten verbunden - insbesondere dann, wenn die beteiligten Datenverarbeitungseinrichtungen Bildschirme oder sonstige Bildanzeigemittel mit abweichenden Formfaktoren aufweisen. Deshalb kann und darf regelmäßig nur der verteilende Benutzer Befunde im System erzeugen, was wiederum eine effektive Zusammenarbeit konterkariert. Ähnliche Probleme treten auch bei herkömmlichen Anwendungen für Online-Konferenzen oder sogenannte Webinare auf.

Der Erfindung liegt daher die Aufgabe zugrunde, ein Verfahren und ein zugehöriges System anzugeben, das eine gemeinsame Auswertung eines medizinischen Bilddatensatzes an mehreren, über ein Datenübertragungsnetz verbundenen Datenverarbeitungseinrichtungen in effizienter Weise unterstützt.

Bezüglich eines Verfahrens wird diese Aufgabe erfindungsgemäß gelöst durch die Merkmale des Anspruches 1. Bezüglich eines Systems wird die obige Aufgabe erfindungsgemäß gelöst durch die Merkmale des Anspruches 5. Vorteilhafte und teils für sich gesehen erfinderische Ausgestaltungsformen und Weiterentwicklungen der Erfindung sind in den Unteransprüchen und der nachfolgenden Beschreibung dargelegt.

Bei den im Zuge des Verfahren bzw. mittels des Systems ausgewerteten Bilddatensätzen kann es sich wahlweise um einfache Bilder mit zweidimensionaler Bildinformation, um Bildstapel mit mehreren jeweils zweidimensionalen Einzelbildern (Schichten), um dreidimensionale Bilddaten (Volumes) oder um Bewegtbildsequenzen mit zweidimensionaler oder dreidimensionaler Bildinformation handeln. Insbesondere ist das System im Rahmen der Erfindung bevorzugt dazu eingerichtet, die Auswertung von Bilddatensätzen verschiedener Art zu unterstützen.

Die Erfindung geht aus von einem Datenverarbeitungs-System mit mindestens zwei Datenverarbeitungseinrichtungen, die über ein Datenübertragungsnetz zum gegenseitigen Datenaustausch verbunden sind. Als "Datenverarbeitungseinrichtung" (nachfolgend kurz "Device") wird allgemein ein mit Bildanzeigemitteln sowie Eingabemitteln ausgestatteter Rechner (Computer) verstanden. Bei mindestens einem der Devices handelt es sich dabei vorzugsweise um einen Personalcomputer oder eine Workstation, der bzw. die mit einem oder mehreren Bildschirmen als Bildanzeigenmittel sowie Tastatur, Maus, etc. als Eingabemittel ausgestattet ist. Alternativ oder zusätzlich ist mindestens eines der Devices durch ein Notebook, einen Tabletcomputer, einen PDA (Personal Digital Assistent) oder ein Smartphone gebildet. Grundsätzlich kann das System in bestimmten Ausführungen der Erfindung ausschließlich gleichartige Devices enthalten. In der Regel umfasst das System aber eine beliebige Kombination von Devices verschiedener Art. Beispielsweise umfasst das System in einer exemplarischen Ausgestaltung als Devices einen Personalcomputer sowie einen Tabletcomputer.

Bei dem Datenübertragungsnetz handelt es sich insbesondere um ein sogenanntes LAN (Local Area Network), das drahtgebundene und/oder drahtlose Datenübertragungsstrecken umfasst. Das Datenübertragungsnetz kann im Rahmen der Erfindung aber auch aus mehreren, auf gleichen oder unterschiedlichen Datenübertragungsstandards beruhenden Teilnetzen zusammengesetzt sein, beispielsweise aus zwei über das Internet verbundenen LANs.

Die im Rahmen des Verfahrens herangezogenen Devices sind hierbei zur Auswertung von medizinischen Bilddatensätzen eingerichtet, indem in jedem dieser Devices mindestens eine Applikation (d.h. Softwareanwendung) implementiert ist, die eine graphische Benutzeroberfläche (auch als "Graphical User Interface", kurz GUI bezeichnet) mit mindestens einem Segment zur Anzeige einer Ansicht eines medizinischen Bilddatensatzes aufweist.

Als "Ansicht" wird hierbei ein digitales, zweidimensionales Bild bezeichnet, das aus einem zugrundeliegenden medizinischen Bilddatensatz abgeleitet wurde und direkt auf dem Bildschirm angezeigt werden kann.

Sofern der zugrundeliegende Bilddatensatz, wie z.B. im Falle eines einfachen Röntgenbilds, eine zweidimensionale Bildinformation aufweist, handelt es sich bei der hieraus abgeleiteten Ansicht beispielsweise um einen hinsichtlich der Pixelauflösung komprimierten, gedrehten, umgefärbten, geglätteten (entrauschten) und/oder in sonstiger Weise bearbeiteten Ausschnitt dieser Bildinformation.

Sofern es sich bei dem zugrundeliegenden Bilddatensatz, wie z.B. im Falle eines üblichen Computertomogramms, um einen Bildstapel handelt, also um eine Mehrzahl von Schichten mit jeweils zweidimensionaler Bildinformation, gibt die daraus abgeleitete Ansicht typischerweise eine (wie vorstehend beschrieben) bearbeiteten Ausschnitt einer bestimmten Schicht dieses Bildstapels wieder. Alternativ kann die Ansicht auch aus einer Kombination (z.B. einer additiven oder subtrahierenden Überlagerung) mehrerer Schichten abgeleitet sein.

Sofern es sich bei dem zugrundeliegenden Bilddatensatz um 3D-Bilddaten (Volumes) handelt, stellt die daraus abgeleitete Ansicht beispielsweise einen Schnitt durch die dreidimensionale Bildinformation des Bilddatensatzes oder eine durch Volume-Rendering erstellte Bildszene dar.

Als "Segment" wird ein Software-Modul bezeichnet, das im Rahmen des GUI zur Anzeige der Ansicht auf einem Bildschirm oder den sonstigen Bildanzeigemitteln des Device dient. Innerhalb des GUI äußert sich das Segment typischerweise in Form eines Rahmens oder Fensters, innerhalb dessen die Ansicht angezeigt wird. Das Segment enthält hierbei typischerweise eine Anzahl von Tools (also Software-Werkzeugen), wie z.B. Drop-Down-Menüs, mittels welcher ein Benutzer in Interaktion mit dem GUI Aktionen zur Manipulation der angezeigten Bilddaten veranlassen kann. Das GUI einer jeden Applikation kann im Rahmen der Erfindung eines oder mehrerer dieser Segmente enthalten. Insbesondere ist vorzugsweise mindestens eine der Applikationen dazu eingerichtet, dass durch Benutzerinteraktion mit dem GUI Segmente in beliebiger Anzahl reversibel geöffnet und wieder geschlossen werden können.

Zur Ableitung der in dem Segment anzuzeigenden Ansicht aus dem zugrundeliegenden Bilddatensatz ist jedem Segment eine Bildbearbeitungs-Pipeline zugeordnet. Als "Bildbearbeitungs-Pipeline" (oder nachfolgend auch kurz "Pipeline") wird allgemein eine Softwarestruktur mit mehreren hintereinander geschalteten Filtern bezeichnet, wobei jeder Filter einen bestimmten Bearbeitungsschritt an den Daten des Bilddatensatzes vornimmt. Als Filter wird demnach ein Softwaremodul bezeichnet, das als Eingangsgröße ursprüngliche oder (von einem ggf. vorgeschalteten Filter) teilbearbeitete Daten des Bilddatensatzes enthält, und das weiterbearbeitete Daten des Bilddatensatzes zur Anzeige an das Segment oder ggf. zur Weiterbearbeitung an einen nachgeschalteten Filter ausgibt.

Beispiele für die Filter der Pipeline sind insbesondere
- ein xy-Filter zur Zentrierung eines anzuzeigenden Bildausschnitts bezüglich einer Bildfläche des zugrundeliegenden Bilddatensatzes,
- ein Zoom-Filter zur Vergrößerung oder Verkleinerung des als Ansicht anzuzeigenden Bildausschnitts,
- ein Farbfilter zur Umfärbung der Bildinformation - unter den Begriff "Farbfilter" werden im Rahmen dieser Anmeldung dabei auch anatomische Filter, wie z.B. auch Knochenfilter oder Weichteilfilter, subsummiert, die bestimmte Farbwertbereiche der Bildinformation, die erfahrungsgemäß bestimmten anatomischen Strukturen, wie z.B. Weichteilgewebe oder Knochen entsprechen, aus der Bildinformation eliminieren oder in dieser hervorheben, und
- ein Rotationsfilter, der die Bildinformation innerhalb der Bildfläche um einen vorgegebenen Winkel dreht.

Filter der vorstehend beschriebenen Arten (insbesondere Farbfilter) können dabei auch mehrfach in der Pipeline vertreten sein. Des Weiteren können auch mehrere der vorstehend beschriebenen Funktionen (z.B. xy-Zentrierung und Zoom) in einem Filter kombiniert sein.

Vorzugsweise ist die Pipeline dabei derart ausgebildet, dass einzelne Filter der Pipeline zur Laufzeit der Applikation automatisch oder durch Benutzerinteraktion mit dem GUI in die Pipeline eingegliedert oder aus der Pipeline ausgegliedert werden können.

Durch die Pipeline wird somit die Ansicht aus den zugrundeliegenden Bilddaten in einem mehrstufigen Prozess erzeugt, indem die Bilddaten die hintereinander geschalteten Filter durchlaufen und hierbei sukzessive weiterbearbeitet werden.

Im Zuge des erfindungsgemäßen Verfahrens werden nun innerhalb des vorstehend beschriebenen Systems in einem ersten Device eine erste Applikation, und in einem zweiten Device eine zweite Applikation ausgeführt. Aus der (Bildbearbeitungs-) Pipeline des Segments (oder eines von ggf. mehreren Segmenten) der ersten Applikation werden hierbei teilbearbeitete Daten des auszuwertenden Bilddatensatzes ausgekoppelt und an die zweite Applikation übermittelt. Das zugehörige Segment der ersten Applikation ist nachfolgend als "Präsentatorsegment" bezeichnet. Die Bildbearbeitungs-Pipeline dieses Präsentatorsegments, aus der die teilbearbeiteten Daten ausgekoppelt werden, ist nachfolgend entsprechend als "Präsentator-Pipeline" bezeichnet.

Die ausgekoppelten Daten werden von der zweiten Applikation empfangen und dort zur Fertigstellung der Ansicht in die Bildbearbeitung-Pipeline des dortigen Segments (oder eines von ggf. mehreren Segmenten der zweiten Applikation) eingekoppelt. Das betreffende Segment der zweiten Applikation ist nachfolgend auch als "Betrachtersegment" bezeichnet. Die Bildbearbeitungs-Pipeline des Betrachtersegments, in die die teilbearbeiteten Daten eingekoppelt werden, ist als "Betrachter-Pipeline" bezeichnet.

Der vorstehend beschriebene Auskopplungs- und Einkopplungsprozess ist dahingehend realisiert, dass im Verlauf des durch die Präsentator-Pipeline durchgeführten, mehrstufigen Bearbeitungsprozesses eine Kopie der teilbearbeiteten Daten erzeugt wird, die in der Betrachter-Pipeline - ggf. anstelle anderer dort bisher verarbeiteter Daten - zu der in dem Betrachtersegment angezeigten Ansicht weiterbearbeitet wird.

Andererseits werden die teilbearbeiteten Daten auch in der Präsentator-Pipeline zur Erzeugung der in dem Präsentatorsegment anzuzeigenden Ansicht weiterbearbeitet.

Mit anderen Worten wird der mehrstufige Bearbeitungsprozess zur Erzeugung der Ansichten für das Präsentatorsegment und für das Betrachtersegment in einem ersten Abschnitt eingleisig in der Präsentator-Pipeline durchgeführt und verzweigt dann in einen ersten Bearbeitungszweig, der in der Präsentator-Pipeline fortgeführt wird und zur Erzeugung der Ansicht für das Präsentatorsegment führt, sowie in einen zweiten Bearbeitungszweig, der in der Betrachter-Pipeline durchgeführt wird und zur Erzeugung der Ansicht für das Betrachtersegment führt.

Durch diese Kopplung der Präsentator-Pipeline mit der Betrachter-Pipeline kann auf einfache Weise sichergestellt werden, dass verschiedenen Benutzern, die entfernt voneinander an verschiedenen Devices arbeiten, die gleiche Bildinformation zur Verfügung gestellt wird, indem bestimmte Bildmanipulationsschritte lediglich einfach in dem ersten Abschnitt der Präsentator-Pipeline durchgeführt werden. Andererseits bietet die sich verzweigende Bearbeitungsstruktur hinreichende Flexibilität, um die zu erstellende Ansicht in einer an die technischen Randbedingungen des jeweiligen Device angepassten Weise zu erstellen.

So werden die teilbearbeiteten Daten des Bilddatensatzes vor der Durchführung einer (präsentatorseitigen) Formfaktoranpassung dieser Daten aus der Präsentator-Pipeline ausgekoppelt, und auch vor einer (betrachterseitigen) Formfaktoranpassung in die Betrachter-Pipeline eingekoppelt.

Als "Formfaktor" wird hierbei eine Information bezeichnet, die die Bildauflösung des Bildschirms oder der sonstigen Bildanzeigemittel des jeweiligen Devices kennzeichnet. Der Formfaktor umfasst insbesondere Angaben zu der Zeilen- und Spaltenlänge der Anzeige, also zu der Anzahl der horizontal bzw. vertikal angezeigten Pixel sowie optional eine Angabe zu der anzeigbaren Farbauflösung für jedes Pixel. Zusätzlich alternativ hiervon kann der Formfaktor allerdings auch in anderer Weise angegeben sein, beispielsweise durch Angabe des Längen-Höhe-Verhältnisses des Bildes (z.B. in Form der Angabe "4:3") und/oder durch Angabe der Länge der Bildschirmdiagonale, etc.

Als "Formfaktoranpassung" wird in diesem Sinne ein Bearbeitungsschritt bezeichnet, durch den vorbearbeitete Daten eines Bilddatensatzes an den individuellen Formfaktor eines bestimmten Device angepasst werden. So müssen beispielsweise die Bilddaten für eine zur Anzeige auf einem Smartphone oder einem Tabletcomputer bestimmten Ansicht im Zuge der Formfaktoranpassung regelmäßig verhältnismäßig stark komprimiert (d.h. hinsichtlich der Pixelauflösung herunterskaliert) werden. Die Formfaktoranpassung umfasst regelmäßig selbst mehrere Bearbeitungsschritte.

In besonders bevorzugter Ausführung der Erfindung werden alle Bearbeitungsschritte, die die Formfaktoranpassung der Daten des Bilddatensatzes betreffen, nach der Auskopplung der teilbearbeiteten Daten aus der Präsentator-Pipeline vorgenommen. Die Formfaktoranpassung erfolgt somit für die Präsentator-Pipeline und die Betrachter-Pipeline jeweils separat. Alle medizinisch relevanten Bearbeitungsschritte, insbesondere die Auswahl des anzuzeigenden Bildausschnitts, optionale Schritte wie Umfärbung, Glättung, Farbselektion, etc. werden dagegen bevorzugt in der Präsentations-Pipeline vor der Auskopplung der teilbearbeiteten Daten vorgenommen.

In an sich üblicher Weise sind den eigentlichen Bilddaten des Bilddatensatzes weitere Daten anderer Datenarten (Nicht-Bilddaten) zugeordnet. Diese Nicht-Bilddaten umfassen insbesondere Metadaten, die Informationen über den Patienten, den Aufnahmezeitpunkt, die für die Aufnahme verwendete bildgebende Modalität sowie die verwendeten Aufnahmeparameter enthalten. Die Metadaten werden bei der Erzeugung der Ansicht üblicherweise vollständig oder zum Teil als Bildtext (als Bildinformation, die einem alphanumerischen Informationsgehalt wiedergibt) den eigentlichen Bilddaten überblendet. Typischerweise wird hierbei durch die Bildbearbeitungs-Pipeline in einem Eckbereich der Ansicht ein Textfeld erzeugt, das eine Auswahl der dem Bilddatensatz zugeordneten Metadaten wiedergibt, wobei dieses Textfeld der eigentlichen Bildinformation überblendet wird.

Zusätzlich oder alternativ umfassen die Nicht-Bilddaten Befunde, die während des Auswertungsprozesses des Bilddatensatzes durch Benutzerinteraktion mit dem GUI einer Applikation erzeugt werden. Der oder jeder Befund umfasst typischerweise eines oder mehrere Markierungsobjekte, wie z.B. einen Kreis, Pfeil, etc. sowie einen von dem Benutzer erzeugten Beschreibungstext. Üblicherweise werden diese Befunde - anders als die Metadaten - nicht unmittelbar in dem Bilddatensatz hinterlegt, sondern in einer von diesem getrennten Datei, die mit dem Bilddatensatz datentechnisch verknüpft ist. Bei der Erstellung der Ansicht werden die Befunde, ähnlich wie Metadaten, der eigentlichen Bildinformation überlagert.

Im Zuge des erfindungsgemäßen Verfahrens werden die Bilddaten des Bilddatensatzes und die Nicht-Bilddaten (also die Daten der mindestens einer weiteren Datenart, insbesondere Metadaten und/oder Befunde) separat voneinander, d.h. vor ihrer Überlagerung, aus der Präsentator-Pipeline ausgekoppelt und an die zweite Applikation übertragen. Ebenso werden die Bilddaten und die Nicht-Bilddaten separat voneinander in die Betrachter-Pipeline eingekoppelt und erst dort zu der Ansicht stets kombiniert (d.h. überlagert).

Die getrennte Übertragung von Bilddaten einerseits und Metadaten und/oder Befunden andererseits ermöglicht auf einfache und effektive Weise, die Nicht-Bilddaten auf verschiedenen Devices mit unterschiedlichen Formfaktoren stets lesbar darzustellen. Hierin unterscheidet sich das vorliegende Verfahren entscheidend von einer reinen Remote-Screen-Funktion, bei der alphanumerische Bestandteile der Ansicht in gleicher Weise vergrößert und verkleinert werden wie die Bilddaten und somit häufig unlesbar werden.

In einer vorteilhaften Ausführung der Erfindung ist vorgesehen, dass auch in dem Betrachtersegment Befunde zu der dort angezeigten Ansicht erzeugt werden können. Verfahrensgemäß wird ein solcher, betrachterseitig erzeugter Befund von der zugeordneten, zweiten Applikation an die erste Applikation übertragen und dort in die Präsentator-Pipeline eingespeist.

Wie vorstehend erwähnt, umfassen das GUI der ersten Applikation und/oder der zweiten Applikation optional mehrere Segmente der vorstehend beschriebenen Art. Verfahrensgemäß ist hierbei vorzugsweise vorgesehen, dass eines dieser Segmente durch Benutzerinteraktion mit dem GUI als Präsentatorsegment auswählbar ist, wobei infolge dieser Auswahl teilbearbeitete Daten des Bilddatensatzes in der vorstehend beschriebenen Weise aus der zugeordneten Bildbearbeitungs-Pipeline ausgekoppelt und an die zweite Applikation übermittelt werden.

Alternativ oder zusätzlich hierzu ist das GUI der zweiten Applikation derart eingerichtet, dass durch Benutzerinteraktion mit dem GUI mindestens eines der dortigen Segmente als Betrachtersegment auswählbar ist.

Vorzugsweise ist das GUI der ersten Applikation oder der zweiten Applikation dabei derart gestaltet, dass jedes der dortigen Segmente als Präsentatorsegment bzw. Betrachtersegment auswählbar ist, optional mit der Einschränkung, dass zu einem Zeitpunkt jeweils nur ein einziges Segment als Präsentator- bzw. Betrachtersegment ausgewählt sein kann. Zweckmäßigerweise ist die Auswahl eines Segments als Präsentator- bzw. Betrachtersegment reversibel, kann also durch Benutzerinteraktion mit dem jeweiligen GUI wieder rückgängig gemacht werden.

In besonders bevorzugter Ausführung der Erfindung ist das GUI mindestens einer Applikation derart gestaltet, dass darin sowohl mindestens ein Präsentatorsegment als auch mindestens ein Betrachtersegment ausgebildet ist oder durch Benutzerinteraktion mit dem GUI als Präsentator- bzw. Betrachtersegment auswählbar ist. Insbesondere ist die Benutzeroberfläche in diesem Fall vorzugsweise derart gestaltet, dass jedes der dortigen Segmente durch Benutzerinteraktion mit der Benutzeroberfläche wahlweise als Präsentatorsegment oder Betrachtersegment auswählbar ist. Eine solche Applikation kann im Zuge des erfindungsgemäßen Verfahrens somit sowohl als "erste Applikation" als auch als "zweite Applikation" im Sinne der vorstehenden Ausführungen betrieben werden.

Erfindungsgemäß ist das vorstehend beschriebene System zur Durchführung des vorstehend beschriebenen, erfindungsgemäßen Verfahrens eingerichtet. Im Rahmen des Systems ist die erste Applikation somit dazu eingerichtet, teilbearbeitete Daten des Bilddatensatzes aus der (Präsentator)-Pipeline des zugehörigen (Präsentator-)Segments auszukoppeln und an die zweite Applikation zu übermitteln. Ebenso ist die zweite Applikation dazu eingerichtet, die übermittelten, teilbearbeiteten Daten des Bilddatensatzes zu empfangen und zur Fertigstellung der Ansicht in die (Betrachter-)Pipeline des zugehörigen (Betrachter-)Segments einzukoppeln.

Die vorstehend beschriebenen vorteilhaften Ausgestaltungen des Verfahrens sind in entsprechender Weise jeweils optional, aber bevorzugt als funktionale Merkmale des Systems implementiert.

So ist die erste Applikation vorzugsweise dazu eingerichtet, die Bilddaten des Bilddatensatzes und zugeordnete Nicht-Bilddaten separat voneinander aus der Präsentator-Pipeline auszukoppeln. Ebenso ist die zweite Applikation hierbei dazu eingerichtet, die Bilddaten und die Nicht-Bilddaten separat voneinander in die Betrachter-Pipeline einzukoppeln und dort zu der Ansicht zu kombinieren.

Des Weiteren ist die erste Applikation vorzugsweise dazu eingerichtet, die teilbearbeiteten Daten des Bilddatensatzes vor Durchführung einer Formfaktoranpassung dieser Daten aus der Präsentator-Pipeline auszukoppeln und ebenfalls vor einer Formfaktoranpassung in die Betrachter-Pipeline einzukoppeln.

In vorteilhafter Ausführung ist die zweite Applikation dazu eingerichtet, dass durch Benutzerinteraktion mit dem zugehörigen GUI in dem Betrachtersegment ein Befund erzeugbar ist, und diesen Befund an die erste Applikation zu übertragen. Die erste Applikation ist hierbei dazu eingerichtet, diesen Befund vor der Auskopplung der teilbearbeiteten Daten in die Präsentator-Pipeline einzuspeisen.

In einer besonders bevorzugten Ausführung umfasst das System zusätzlich zu der ersten Applikation und der mindestens einen zweiten Applikation eine zentrale Kollaborationseinheit, die die vorstehend beschriebene Datenübermittlung zwischen diesen Applikationen vermittelt. Diese zentrale Kollaborationseinheit ist hierbei vorzugsweise in einer Cloud, insbesondere einer sogenannten Public Cloud implementiert.

Als "Cloud" (Rechnerwolke) wird dabei eine Datenverarbeitungseinrichtung verstanden, die von einem von dem Benutzer unabhängigen Cloud-Betreiber ("Cloud Vendor") zur Verfügung gestellt und betrieben wird. Der "Cloud Vendor" stellt hierbei dem Benutzer die Hardware und gegebenenfalls die Software der Cloud im Rahmen eines Nutzungsvertrags (Subscription) als Dienst zur Verfügung.

Je nach dem Benutzerkreis, an den die jeweilige Cloud adressiert ist, unterscheidet man zwischen
- einer sogenannten "Public Cloud", deren Dienste von jedem in Anspruch genommen werden können, und
- einer sogenannten "Privat Cloud", die nur Benutzern einer bestimmten Organisation, insbesondere eines bestimmten Konzerns zugänglich ist.

Für jeden Benutzer einer Public Cloud sind die Zugriffsberechtigungen auf bestimmte Hardware- und Softwarebestandteile der Cloud durch die dem Benutzer zugeordnete Subscription geregelt. Public Clouds sind hierdurch insbesondere "mandantenfähig" (multi-tenant). Dies bezeichnet die Fähigkeit, Daten, Benutzerverwaltung und Rechenoperationen für Benutzer mit verschiedener Subscription strikt getrennt zu halten. Ein Benutzer der Public Cloud kann also in die Daten, Benutzerverwaltung und Rechenoperationen eines anderen Benutzers mit unterschiedlicher Subscription keinen Einblick nehmen.

In einer bevorzugten Weiterbildung der Erfindung sind die Applikationen - wie bei einer herkömmlichen Konferenzumgebung - vorzugsweise auch zur gegenseitigen Übertragung von Tonsignalen (insbesondere aufgenommener Sprache) sowie optional Videosignalen eingerichtet. Auch die Ton- bzw. Videoübertragung wird hierbei zweckmäßigerweise über die zentrale Kollaborationseinheit vermittelt.

Die mit der Erfindung verbundenen Vorteile bestehen insbesondere darin, dass der Betrieb des Systems nach dem erfindungsgemäßen Verfahren ein nur vergleichsweise geringes Datenübertragungsaufkommen beansprucht, zumal lediglich die bereits teilbearbeiteten Bilddaten sowie ggf. Nicht-Bilddaten wie Metadaten und/oder Befunde zwischen den Applikationen übertragen werden, nicht aber ganze Bilddatensätze. Somit werden insbesondere nennenswerte Latenzzeiten für die Datenübertragung effektiv vermieden. Weiterhin wird durch die Erfindung ermöglicht, entsprechende Bildinformation auf mehreren Devices mit jeweils hinsichtlich der technischen Gegebenheiten des jeweiligen Device optimierter Qualität anzuzeigen.

Ein entscheidender Vorteil des Systems und des Verfahrens ist zudem, dass verschiedene Applikationen im Wesentlichen gleiche Ansichten des Bilddatensatzes anzeigen können, auch wenn die zur Erzeugung dieser Ansichten erforderlichen Bildmanipulationsfunktionen nicht in jeder der zusammenarbeitenden Applikationen vorhanden sind. Vielmehr genügt es, wenn die benötigten Funktionen in der Präsentator-Pipeline vorhanden sind. Auf diese Weise können beispielsweise Ansichten von Mammographie-Datensätzen, die unter Nutzung von mammographiespezifischen Datenmanipulations-Algorithmen der Präsentator-Pipeline aufbereitet wurden, auch in Betrachtersegmenten anderer Applikationen angezeigt werden, die selbst über diese Algorithmen nicht verfügen. Des Weiteren ist der Einsatz des Verfahrens und das zugehörigen Systems auch für die Planung und Durchführungen von Notfall-Behandlungen mit Unterstützung durch bildgebende Untersuchungsmethoden, insbesondere Computertomographie, besonders vorteilhaft, zumal in solchen Fällen das für die Auswertung der Bilddaten erforderliche Einsatzteam häufig aus Zeitgründen nicht an dem Einsatzort versammelt werden kann.

Nachfolgend werden Ausführungsbeispiele der Erfindung anhand einer Zeichnung näher erläutert. Darin zeigen:

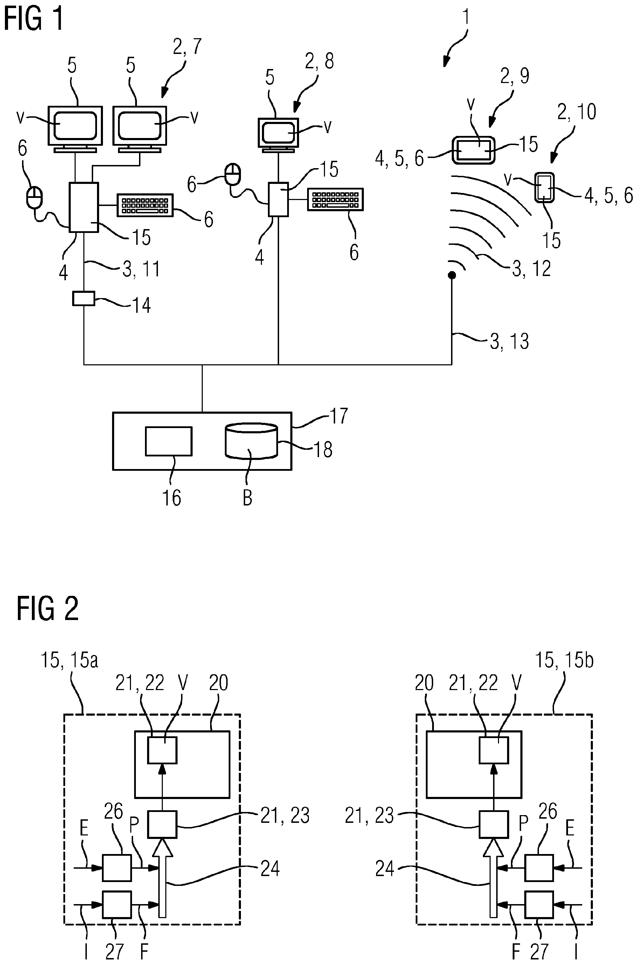

- FIG 1: in einem schematischen Blockschaltbild ein System zur gemeinsamen Auswertung eines medizinischen Bilddatensatzes durch mehrere Benutzer mit vier Datenverarbeitungsgeräten (nachfolgend kurz als "Devices" bezeichnet), einem diese Devices verbindenden Datenübertragungsnetz sowie einem Datenspeicher zur Speicherung von medizinischen Bilddatensätzen und einer zentralen Kollaborationseinheit, wobei der Datenspeicher und die Kollaborationseinheit in einer Public Cloud implementiert sind, und wobei in jedem Device eine Applikation zur Ableitung und Anzeige einer aus dem Bilddatensatz abgeleiteten Ansicht implementiert ist,
- FIG 2: in einem schematischen Blockschaltbild zwei der genannten Applikationen, wobei jede Applikation jeweils eine graphische Benutzeroberfläche mit einem Segment zur Anzeige der Ansicht, eine zugeordnete (Bildbearbeitungs-)Pipeline zur Ableitung der Ansicht aus dem zugrundeliegenden Bilddatensatz, einen Event-Handler zur Beeinflussung der durch die Pipeline vorgenommenen Bildmanipulation sowie ein Befundverwaltungsmodul zur Verwaltung von Befunden aufweist, und wobei die beiden Applikationen in einem an sich herkömmlichen Stand-Alone-Betrieb betrieben, in dem jede Applikation isoliert, ohne Wechselwirkung mit der jeweils anderen Applikation arbeitet,
- FIG 3: in einem schematischen Blockschaltbild in größerem Detail eine der Bildbearbeitungs-Pipelines, den Event-Handler und das Befundverwaltungsmodul in dem Stand-Alone-Betrieb,
- FIG 4: in Darstellung gemäß FIG 2 die dortigen Applikationen in einem Kollaborationsbetrieb, in dem zwischen den Applikationen zur gemeinsamen Auswertung eines medizinischen Bilddatensatzes Daten über die zentrale Kollaborationseinheit ausgetauscht werden,
- FIG 5: in Darstellung gemäß FIG 3 die BildbearbeitungsPipelines der beiden Applikationen in dem Kollaborationsbetrieb, und
- FIG 6: in einem grob vereinfachten Schema die Struktur der die Kollaboration zwischen den Applikationen ermöglichenden Komponenten.

Einander entsprechende Teile, Größen und Strukturen sind nachfolgend in allen Figuren stets mit gleichen Bezugszeichen versehen.

FIG 1 zeigt in grober schematischer Vereinfachung ein System 1 zur gemeinsamen Auswertung von medizinischen Bilddatensätzen B.

Das System 1 umfasst hardwareseitig eine Mehrzahl von Datenverarbeitungseinrichtungen, die nachfolgend kurz als Devices 2 bezeichnet sind, und die zum gegenseitigen Datenaustausch über ein (Datenübertragungs-)Netz 3 miteinander verbunden sind.

Jedes Device 2 umfasst allgemein einen Rechner 4 mit angeschlossenen Bildanzeigemitteln 5 (insbesondere einem oder mehreren Bildschirmen) sowie Eingabemitteln 6 für Benutzerinteraktionen mit dem jeweiligen Device 2, wie z.B. einer Tastatur oder einer (Computer-)Maus.

In dem Beispiel gemäß FIG 1 sind exemplarisch vier verschiedenartige Devices 2 dargestellt, nämlich
- eine Befundungsstation 7, bei der der Rechner 4 beispielsweise durch eine leistungsstarke Workstation gebildet ist, und die als Bildanzeigemittel 5 zwei großformatige Bildschirme umfasst,
- einen PC-Arbeitsplatz 8, bei der der Rechner 4 durch einen gewöhnlichen Desktop-Personalcomputer (PC), und die Bildanzeigemittel 5 durch einen gewöhnlichen Computermonitor gebildet sind,
- einen Tabletcomputer 9, bei dem der Rechner 4, die Bildanzeigemittel 5 und die Eingabemittel 6 in einem tragbaren Kompaktgerät mit Touch-Display integriert sind, sowie
- ein Smartphone 10, bei dem der Rechner 4, die Bildanzeigemittel 5 und die Eingabemittel 6 ebenfalls in einem - im Vergleich zu dem Tabletcomputer 9 aber kleinformatigeren - tragbaren Kompaktgerät mit Touch-Display integriert sind.

Alternativ oder zusätzlich zu den dargestellten Devices 2 kann das System 1 auch andersartige Datenverarbeitungseinrichtungen, z.B. ein oder mehrere Notebooks, umfassen. Generell kann das System 1 im Rahmen der Erfindung eine beliebige Anzahl von Devices 2, mindestens jedoch zwei Devices 2 umfassen.

Die Devices 2 sind in dem betriebsfähigen Zustand des Systems 1 in der Regel räumlich getrennt voneinander angeordnet, wobei grundsätzlich eine beliebige, weltweit verstreute Anordnung der Devices 2 möglich ist. Beispielsweise bildet die Befundungsstation 7 einen Bestandteil einer IT-Infrastruktur einer medizinischen Einrichtung, insbesondere einer Klinik, während der PC-Arbeitsplatz 8 ein im privaten Bereich eines Benutzers angeordneter Heimarbeitsplatz ist, und während der Tabletcomputer 9 und das Smartphone 10 als mobile Geräte an wechselnden Standorten betrieben werden.

Entsprechend der jeweiligen räumlichen Anordnung der Devices 2 besteht das Netz 3 in bevorzugter Ausführung des Systems 1 aus einem einzelnen, einheitlichen Netz oder einer Mehrzahl von zusammenwirkenden Teilnetzen. In dem vereinfachten Beispiel gemäß FIG 1 umfassen die Teilnetze des Netzes 3 insbesondere
- ein drahtgebundenes LAN 11 der medizinischen Einrichtung, in der die Befundungsstation 7 angeordnet ist,
- ein Drahtlosnetzwerk 12 (z.B. ein WLAN oder ein Mobilfunknetz), durch welches der Tabletcomputer 9 und das Smartphone 10 an das System 1 angebunden sind, sowie
- das Internet 13, durch welches die anderen Teilnetze miteinander verbunden sind.

Lokale Teilnetze wie das LAN 11 sind hierbei typischerweise durch eine exemplarisch angedeutete Firewall 14 mit dem Internet 13 verbunden.

Softwareseitig umfasst das System 1 eine Anzahl von Applikationen (d.h. Software-Anwendungen) 15 zur Anzeige und Bearbeitung der Bilddatensätze B, wobei jeweils mindestens eine der Applikationen 15 in jedem der Devices 2 lauffähig implementiert ist. Grundsätzlich können hierbei gleiche Applikationen 15 in allen Devices 2 implementiert sein. In der bevorzugten Realisierung des Systems 1 sind auf den einzelnen Devices 2 aber unterschiedliche Applikationen 15 oder unterschiedliche Varianten einer Applikation 15 implementiert, die jeweils einen an einen spezifischen medizinischen Anwendungsbereich und/oder an das jeweils zugeordnete Device 2 angepassten Funktionsumfang aufweisen.

Beispielsweise ist die in der Befundungsstation 7 implementierte Applikation 15 durch eine Vollversion einer spezialisierten Anwendung zur Anzeige und Befundung von Mammographie-Bildern gebildet, während in dem PC-Arbeitsplatz 8 beispielsweise eine umfangsreduzierte Version der gleichen Applikation 15 implementiert ist, bei der numerisch aufwändige Funktionen der Vollversion fehlen. In dem Tabletcomputer 9 und dem Smartphone 10 sind dagegen als Applikationen 15 beispielsweise lediglich generische Anzeigeprogramme für Bilddaten (Multi-Modality-Reader) ohne spezialisierte Funktionen zur Bildbearbeitung und Bildauswertung implementiert.

Zusätzlich zu den Applikationen 15 umfasst das System 1 softwareseitig eine zentrale Kollaborationseinheit 16, die eine Zusammenarbeit zwischen den einzelnen Applikationen 15 koordiniert. In der bevorzugten Ausbildung des Systems 1 ist die Kollaborationseinheit 16 in einer (Public) Cloud 17, insbesondere in dem Cloud-Dienst "Azure" der Fa. Microsoft implementiert. Die Cloud 17 selbst ist hierbei im engeren Sinne kein Teil des Systems 1, sondern wird von diesem nur genutzt.

Ferner umfasst das System 1 einen Speicher 18, in dem die Bilddatensätze B hinterlegt sind. In dem Beispiel gemäß FIG 1 ist der Speicher 18 ebenfalls in der Cloud 17 angeordnet. Grundsätzlich können die Bilddatensätze B zusätzlich oder alternativ auch am Ort einer der Devices 2 hinterlegt sein, beispielsweise in der medizinischen Einrichtung, in der die Befundungsstation 7 angeordnet ist. Der Speicher 18 kann insbesondere auch aus mehreren Einzelspeichern bestehen, die an einem Ort oder verteilt an mehreren Orten angeordnet sind. In dem Speicher 18 sind insbesondere Bilddatensätze B unterschiedlicher Art hinterlegt, insbesondere Einfachbilder (Bilddatensätze B mit einfach zweidimensionaler Bildinformation, Bildstapel und Volumes). Lediglich aus Vereinfachungsgründen wird im Folgenden angenommen, dass es sich bei dem auszuwertenden Bilddatensatz B um ein Einfachbild, beispielsweise eine Mammographieaufnahme handelt.

FIG 2 zeigt schematisch und in grober Vereinfachung den Aufbau zweier Applikationen 15a und 15b, bei denen es sich um beliebige der im Zusammenhang mit FIG 1 gezeigten Applikationen 15 handeln kann.

Wie aus der Darstellung erkennbar ist, umfasst jede der beiden Applikation 15a,15b eine graphische Benutzeroberfläche, die nachfolgend kurz als GUI 20 bezeichnet ist. Als Bestandteil des GUI 20 umfassen die Applikationen 15a,15b jeweils eines oder mehrere Segmente 21, von denen jedes jeweils zur Anzeige einer Ansicht V des auszuwertenden Datensatzes B dient, und von denen in FIG 2 lediglich aus Gründen der Übersichtlichkeit jeweils nur eines explizit dargestellt ist.

In dem GUI 20 äußert sich jedes Segment 21 in einem rechteckigen Rahmen oder Fenster, in dem die Ansicht V angezeigt wird. Jedes Segment 21 enthält hierbei eine Anzahl von Drop-Down-Menus, die der Benutzer durch Interaktion mit dem GUI 20 (insbesondere Tippen auf eine korrespondierende Schaltfläche mit der Maus oder einem vergleichbaren Eingabemittel oder durch Vollführen einer bestimmten Mauszeigerbewegung) aktivieren kann. Über diese Drop-Down-Menüs kann der Benutzer durch Interaktion mit dem GUI 20 Aktionen zur Manipulation der angezeigten Bilddaten veranlassen.

Die Anzahl der in dem GUI 20 nebeneinander positionierten Segmente 20 ist hierbei benutzerspezifisch bestimmt. Hierzu kann der Benutzer durch Interaktion mit dem GUI 20 beliebig viele Segmente 21 öffnen und schließen.

In einer beispielhaften Ausführung der Applikation 15 in einer Client-Server-Umgebung ist aus technischen Gründen vorzugsweise jedes Segment 21 aus einem Frontend 22 und einem Backend 23 gebildet, wobei das Frontend 22 die graphischen Bedienelemente des Segments 21, insbesondere also die Bildfläche und die genannten Drop-Down-Menüs definiert, während das Backend 23 die mit diesen Bedienelementen verknüpfte Programm-Logik enthält. In einer alternativen Ausführung, wie sie insbesondere auf dem Tabletcomputer 9 und dem Smartphone 10 implementiert ist, ist die Funktionalität des Frontends 22 und des Backends 23 in einer einzigen Softwarekomponente zusammengefasst.

Des Weiteren enthalten die Applikationen 15a,15b zu jedem Segment 21 jeweils eine zugeordnete (Bildbearbeitungs-)Pipeline 24, die zur Ableitung der in dem jeweiligen Segment 21 darzustellenden Ansicht V aus dem zugrundeliegenden Bilddatensatz B dient.

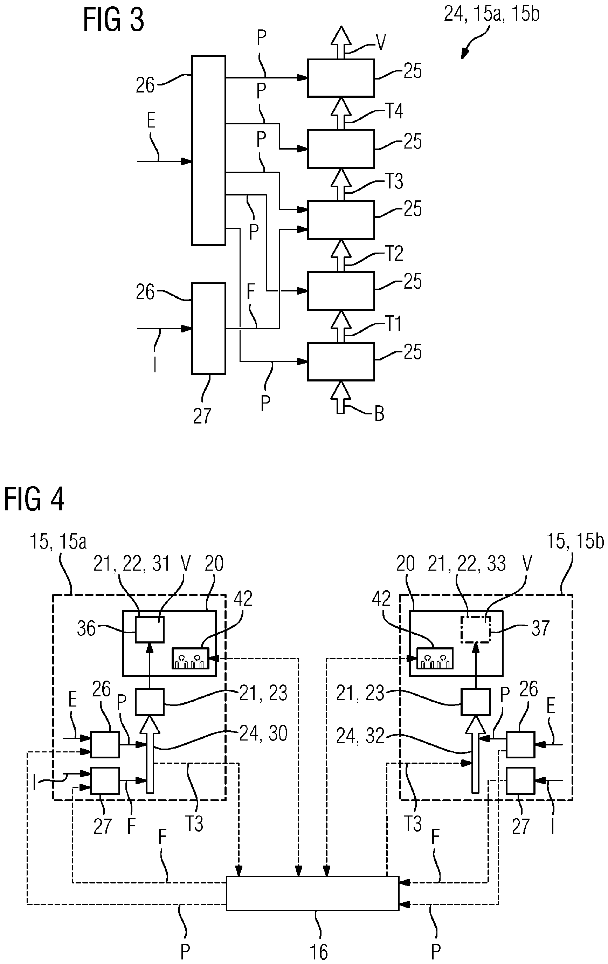

Wie in FIG 3 schematisch und grob vereinfacht angedeutet ist, umfasst jede Pipeline 24 jeweils mehrere hintereinander geschaltete Filter 25, von denen jeder einen bestimmten Bearbeitungsschritt an den Daten des Bilddatensatzes B vornimmt, um aus dem ursprünglichen Bilddatensatz B die Ansicht V zu erstellen.

In FIG 3 sind lediglich beispielhaft fünf Filter 25 dargestellt, bei denen es sich insbesondere - in der Reihenfolge der Bildbearbeitung - um
- einen Filter 25 zur Auswahl des anzuzeigenden Bildausschnitts der ursprünglichen Bilddaten des Bilddatensatzes (umfassend xy-Zentrierung des Bildmittelpunkts und Zoom),
- einen Filter 25 zur Extraktion der anzuzeigenden Metadaten aus dem Bilddatensatz und Verteilung dieser Metadaten auf der Bildfläche,
- einen Filter 25 zur Auswahl von dem Bildausschnitt zugehörigen, hinterlegten Befunden F und zur Platzierung dieser Befunde auf der Bildfläche,
- einen Filter 25 zur Anpassung der Daten des Bilddatensatzes inklusive der extrahierten Befunde F an den Formfaktor des jeweiligen Devices 2 sowie
- einen Filter 25 zur Kombination der ausgewählten Bilddaten, Metadaten und Befunde F zu der Ansicht V
handelt.

Die im Zusammenhang mit den fünf vorstehend genannten Filtern 25 beschriebenen Funktionen können auch auf mehr als fünf Filter 25 verteilt sein oder in anderer Form zusammengefasst sein. Des Weiteren kann die Pipeline 25 zahlreiche weitere Filter umfassen.

Im Zuge der Formfaktoranpassung werden einerseits die Bilddaten des anzuzeigenden Bildausschnitts an die Bildschirmauflösung des zugehörigen Devices 2 angepasst. Die Bildinformation wird dabei entsprechend hochskaliert oder herunterskaliert, um die Pixel des Bilddatenausschnitts auf eine - regelmäßig abweichende - Anzahl von Bildschirmpixeln abzubilden.

Andererseits wird im Zuge der Formfaktoranpassung aus den extrahierten Metadaten ein entsprechender Metadaten-Bildtext (also eine Bildinformation mit alphanumerischem Informationsgehalt) erzeugt, wobei dieser Bildtext unter Berücksichtigung des Formfaktors des zugehörigen Devices 2 erzeugt wird, um die Lesbarkeit des Bildtextes sicherzustellen.

Schließlich werden im Zuge der Formfaktoranpassung die extrahierten und auf der Bildfläche verteilten Befunde F in ein entsprechendes Befundbild umgewandelt (also eine Bildinformation, die die graphische und alphanumerische Information wiedergibt). Auch bei diesem Teilschritt wird der Formfaktor des zugehörigen Device 2 berücksichtigt, um die Erkennbarkeit der Befunde sicherzustellen.

Im Zuge der Kombination werden der Bildtext und das Befundbild den eigentlichen Bilddaten überlagert. Die fertiggestellte Ansicht V enthält somit den ausgewählten Bildausschnitt des Bilddatensatzes mit einer darin eingeblendeten Metadatenauswahl und den ebenfalls eingeblendeten Befunden F.

Durch die Pipeline 24 wird somit die Ansicht V aus dem zugrundeliegenden Bilddatensatz B in einem mehrstufigen Prozess erzeugt, indem Bilddaten, Metadaten und Befunde F die hintereinander geschalteten Filter 25 durchlaufen und hierbei sukzessive weiterbearbeitet werden. Die einzelnen Filter 25 der Pipeline 24 (mit Ausnahme des letzten Filters 25) geben hierbei jeweils teilbearbeitete Daten T1 bis T4 an den jeweils nachgeschalteten Filter 25 weiter.

Die Pipeline 24 ist derart ausgebildet, dass einzelne Filter 25 zur Laufzeit der Applikation 15a,15b durch Benutzerinteraktion mit dem GUI 20 in die Pipeline 24 eingegliedert oder aus der Pipeline 24 ausgegliedert werden können. Beispielsweise wird ein (nicht explizit dargestellter) Farbfilter zur Färbung der standardmäßig monochromen Bilddaten in die Pipeline 24 eingegliedert, wenn der Benutzer durch Auswahl eines entsprechenden Menüpunktes in dem Segment 21 die Farbdarstellungsfunktion aktiviert. Andere Filter 25 werden automatisch in Abhängigkeit der Art des geladenen Bilddatensatzes B in die Pipeline 24 eingegliedert oder aus der Pipeline 24 ausgegliedert.

Des Weiteren werden bestimmte Eigenschaften von zumindest einigen der Filter 25 durch Parameter P bestimmt, die den Filtern 25 durch einen Event-Handler 26 vorgegeben werden.

Der Event-Handler 26 bestimmt seinerseits die Parameter P in Abhängigkeit von zugeführten Ereignissen E, die entweder automatisch durch die Applikation 15a, 15b oder infolge einer Benutzerinteraktion mit dem GUI 20 (z.B. der Betätigung der linken Maustaste bei einer bestimmten Position des Mauszeigers) erzeugt werden. Die Parameter P bestimmen im Falle der vorstehend beschriebenen Filter 25 beispielsweise
- die xy-Position der ursprünglichen Bilddaten, bezüglich der der anzuzeigende Bildausschnitt zu zentrieren ist und einen Zoom-Faktor,
- Optionen zur Auswahl der anzuzeigenden Metadaten, etc.,
- Optionen zur Darstellung der Befunde F,
- Angaben zur Schriftgröße der einzublendenden Metadaten und Befunde F sowie
- Angaben, ob der Metadaten-Bildtext und/oder das Befundbild den Bilddaten überlagert werden sollen.

Zur Verwaltung von bestehenden Befunden F sowie zur Erstellung von neuen Befunden F nach Maßgabe von entsprechenden Eingaben I des die jeweilige Applikation 15a,15b bedienenden Benutzers umfassen die Applikationen 15a,15b zudem jeweils ein Befundverwaltungsmodul 27, wobei dieses Befundverwaltungsmodul 27 die bestehenden und neu erstellten Befunde F in die Pipeline 24 einspeist.

FIG 2 zeigt die vorstehend beschriebenen Applikationen 15a und 15b in einem Betriebsmodus, in dem die Segmente 21 der Applikationen 15 des Systems 1 in an sich herkömmlicher Weise unabhängig voneinander arbeiten. In diesem nachfolgend als "Stand-Alone-Betrieb" bezeichneten Betriebsmodus findet kein Datenaustausch zwischen den Applikationen 15a und 15b und deren Segmenten 21 statt. Insbesondere wird somit in dem "Stand-Alone-Betrieb" durch die Applikationen 15a,15b eine gemeinsame Auswertung des Bilddatensatzes B an mehreren Devices 2 auch nicht unterstützt.

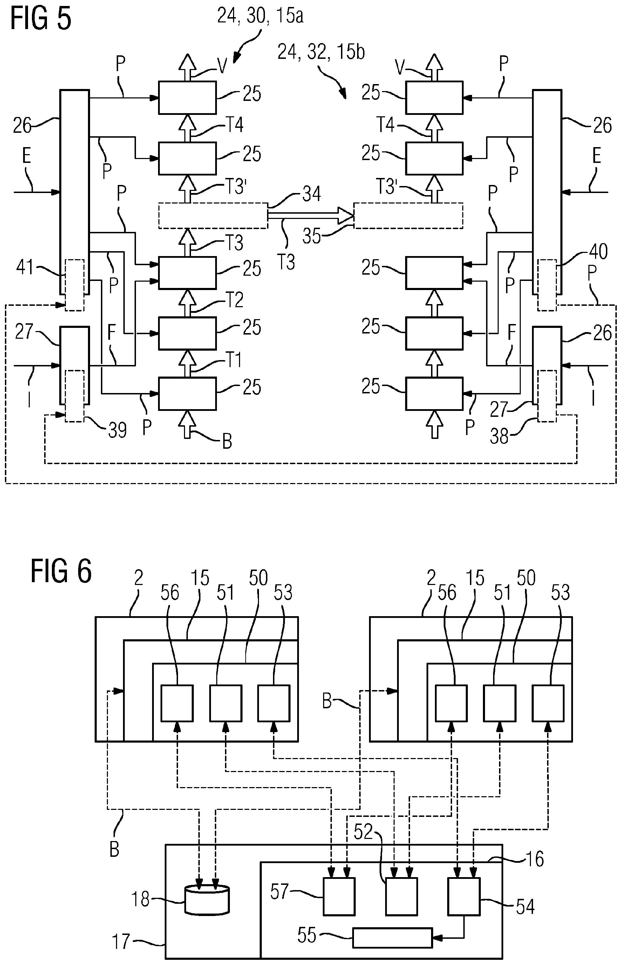

Um dennoch eine effektive gemeinsame Datenauswertung zu ermöglichen, sind die Applikationen 15 des Systems 1 derart eingerichtet, dass einzelne Segmente 21 des GUI 20 reversibel aus dem "Stand-Alone-Betrieb" in einen anderen Betriebsmodus versetzt werden können, der nachfolgend als "Kollaborations-Betrieb" bezeichnet und anhand der FIG 4 und 5 verdeutlicht ist.

Wie aus diesen Figuren erkennbar ist, zeichnet sich der Kollaborationsbetrieb dadurch aus, dass die Pipelines 24 von zwei oder mehr Segmenten 21 verschiedener Applikationen 15a,15b (die sinnvollerweise auf verschiedenen Devices 2 implementiert sind) miteinander gekoppelt sind, indem - gemäß der Darstellung der FIG 4 - aus der Pipeline 24 des Segments 21 einer ersten Applikation 15 (gemäß FIG 4 beispielhaft der Applikation 15a) teilbearbeitete Daten T3 ausgekoppelt und in die Pipeline 24 des Segments 21 mindestens einer zweiten Applikation 15 (gemäß FIG 4 beispielhaft der Applikation 15b) eingekoppelt werden.

Im Sinne der vorstehend eingeführten Konnotation werden die Pipeline 24 der Applikation 15a und das zugehörige Segment 21 somit als Präsentator-Pipeline 30 bzw. Präsentatorsegment 31 betrieben. Die Pipeline 24 der Applikation 15b und das zugehörige Segment 21 werden dagegen als Betrachter-Pipeline 32 bzw. Betrachtersegment 33 betrieben.

Aus FIG 5 ist zu entnehmen, dass die Kopplung der Pipelines 24 in einer bevorzugten Ausführungsform dadurch erzielt wird, dass in die Präsentator-Pipeline 30 und die Betrachter-Pipeline 32 jeweils ein Koppelfilter 34 bzw. 35 geschaltet werden. Der Koppelfilter 34 koppelt hierbei die Daten T3 aus der Präsentator-Pipeline 30 aus und übermittelt diese Daten T3 an die Applikation 15b. Dort werden die Daten T3 durch das Koppelmodul 35 an gleicher Stelle der Bearbeitungsfolge in die Betrachter-Pipeline 32 eingekoppelt.

Andererseits gibt der Koppelfilter 34 teilbearbeitete Daten T3` auch an den innerhalb der Präsentator-Pipeline 30 folgenden Filter 25 ab. Die Daten T3` gleichen hierbei im Wesentlichen den ausgekoppelten Daten T3. Allerdings unterscheiden sich die Daten T3` von den Daten T3 in bevorzugter Ausführung durch eine von dem Koppelmodul 34 hinzugefügte Markierung 36 (vorzugsweise in Form eines in einer bestimmten Farbe, z.B. rot, gehaltenen Rahmens), die als Teil der Ansicht V in dem Präsentatorsegment 31 angezeigt wird und die das Präsentatorsegment 31 somit als solches sichtbar kennzeichnet (FIG 4).

Der Koppelfilter 35 ersetzt die in den vorgeschalteten Filtern 25 der Betrachter-Pipeline 32 bearbeiteten Daten durch die eingekoppelten Daten T3. Die Betrachter-Pipeline 32 wird somit durch die Daten T3 der Präsentator-Pipeline 30 quasi gekapert. Auch der Koppelfilter 35 fügt den Daten T3 in bevorzugter Ausführung eine zusätzliche Markierung 37 hinzu und gibt entsprechend modifizierte Daten T3' an den nachgeschalteten Filter 25 weiter. Diese Markierung 37 (FIG 4) wird vorzugsweise in Form eines in einer anderen Farbe, z.B. gelb, gehaltenen Rahmens erzeugt, der als Teil der Ansicht V in dem Betrachtersegment 33 angezeigt wird und der das Betrachtersegment 33 somit als solches sichtbar kennzeichnet.

Die Kopplungsfilter 34 und 35 sind derart in die Präsentator-Pipeline 30 bzw. in die Betrachter-Pipeline 32 eingesetzt, dass alle medizinisch relevanten Bearbeitungsschritte, insbesondere die Auswahl des zu befundenden Bildausschnitts, die Auswahl der anzuzeigenden Metadaten, die Auswahl der anzuzeigenden Befunde F, ggf. die Umfärbung der Bilddaten, etc. vor der Auskopplung der Daten T3 ausschließlich in der Präsentator-Pipeline 30 vorgenommen werden. Die Formfaktoranpassung wird dagegen nach der Auskopplung der Daten T3 vorgenommen und findet somit separat und unabhängig voneinander sowohl in der Präsentator-Pipeline 30 als auch in der Betrachter-Pipeline 32 statt, wobei dem die Formfaktoranpassung durchführenden Filter 25 in der Präsentator-Pipeline 30 und der Betrachter-Pipeline 32 jeweils in Hinblick an den Formfaktor des zugehörigen Device 2 angepasste Parameter P vorgegeben werden.

Des Weiteren findet auch die Kombination der ausgewählten Bilddaten mit Metadaten und Befunden F, also die Überlagerung der Bilddaten mit Metadaten-Bildtext und einem Befundbild separat und unabhängig voneinander sowohl in der Präsentator-Pipeline 30 als auch in der Betrachter-Pipeline 32 statt. Bilddaten, Metadaten und Befunde F werden somit als voneinander getrennte Bestandteile der teilbearbeiteten Daten T3 separat und unabhängig voneinander aus der Präsentator-Pipeline 30 ausgekoppelt und in die Betrachter-Pipeline 32 eingekoppelt.

Als Ergebnis dieses Prozesses enthalten die in dem Präsentatorsegment 31 und dem Betrachtersegment 33 angezeigten Ansichten V stets die gleiche medizinische Bildinformation, wobei diese Bildinformation jeweils in einer für das jeweilige Device 2 optimierten Qualität dargestellt wird.

Andererseits werden in dem Kollaborationsbetrieb der Applikationen 15a und 15 Befunde F, die das Befundverwaltungsmodul 27 der Applikation 15b aufgrund von Eingaben I des diese Applikation 15b bedienenden Benutzers ("Betrachter") erzeugt, nicht - wie in dem Stand-Alone-Betrieb der Applikation 15b - in die Betrachter-Pipeline 32 eingespeist. Vielmehr werden diese betrachterseitig erzeugten Befunde F an die Applikation 15a übermittelt und durch das dortige Befundverwaltungsmodul 27 verwaltet und in die Präsentator-Pipeline 30 eingespeist. Hierdurch werden die betrachterseitig erzeugten Befunde F sowohl in dem Präsentatorsegment 31 als auch in dem Betrachtersegment 33 sichtbar.

Des Weiteren werden Parameter P, die der Event-Handler 26 der Applikation 15b aufgrund von betrachterseitig auftretenden Ereignissen E erzeugt, nur zum Teil in die Betrachter-Pipeline 32 eingespeist. Zu einem anderen Teil werden diese Parameter P an die Applikation 15a übermittelt und durch den dortigen Event-Handler 26 in die Präsentator-Pipeline 30 eingespeist. Wie in FIG 5 angedeutet ist, werden dabei insbesondere nur solche Parameter P an die Applikation 15a übermittelt, die, die den Koppelfiltern 34 und 35 vorgeschaltete Filter 25 betreffen. In dem vorstehend beschriebenen Beispiel werden also betrachterseitig generierte Parameter P, die medizinisch relevante Bildmanipulationsschritte wie die Auswahl des Bildausschnitts, die Auswahl der Metadaten und die Auswahl der Befunde F betreffen, an die Applikation 15a übermittelt und in die Präsentator-Pipeline 30 eingespeist, wodurch die hierdurch verursachten Auswirkungen auf den Bildmanipulationsprozess sowohl in dem Präsentatorsegment 31 als auch in dem Betrachtersegment 33 sichtbar werden. Auf diese Weise kann somit auch der Betrachter - für alle beteiligten Benutzer sichtbar - den in der Ansicht V dargestellten Bildausschnitt verschieben oder vergrößern/verkleinern. Dagegen werden alle betrachterseitig generierten Parameter P, die den Koppelfiltern 34 und 35 nachgeschaltete Filter 25 betreffen, nur in die Betrachter-Pipeline 32 eingespeist und beeinflussen somit auch nur die in dem zugehörigen Betrachtersegment 33 angezeigte Ansicht V.

Wie in FIG 5 angedeutet ist, wird die Übermittlung der betrachterseitig generierten Befunde F und der betrachterseitig generierten Parameter P an die Applikation 15a durch Einschubmodule 38, 39, 40 und 41 vorgenommen, die im Kollaborations-Betrieb - z.B. nach Art von Plug-Ins - mit dem betrachterseitigen Befundverwaltungsmodul 27, dem präsentatorseitigen Befundverwaltungsmodul 27, dem betrachterseitigen Event-Handler 26 bzw. dem präsentatorseitigen Event-Handler 26 gekoppelt sind.

In alternativer Ausführung des Systems 1 sind separate Einschubmodule 38, 39, 40 und 41 nicht vorgesehen. Stattdessen ist die Funktion dieser Einschubmodule 38-41 als fester (untrennbarer) und zur Laufzeit der Applikationen 15a,15b aktivierbarer und deaktivierbarer Bestandteil der Befundverwaltungsmodule 27 und der Event-Handler 26 der Applikationen 15a,15b implementiert.

Abweichend von dem vorstehend beschriebenen Prozess werden in einer anderen Ausführung des Systems 1 und des damit durchgeführten Verfahrens anstelle von betrachterseitig erzeugten Befunden F und Parametern P unmittelbar die zugrundeliegenden Eingaben I bzw. Ereignisse E der zweiten Applikation 15b an die erste Applikation 15a übertragen. Die Ableitung von Befunden F und Parametern P aus den übertragenen Eingaben I bzw. Ereignisse E erfolgt hierbei präsentatorseitig durch die Applikation 15a.

Jedes Segment 21 der Applikationen 15a,15b kann durch den jeweiligen Benutzer durch Interaktion mit dem GUI 20 reversibel zwischen dem Stand-Alone-Betrieb gemäß FIG 2,3 und dem Kollaborationsbetrieb gemäß FIG 4,5 geschaltet werden. Dem Benutzer wird hierzu als Bestandteil des GUI 20 - ständig oder nach Aktivierung durch den Benutzer - durch die jeweilige Applikation 15a,15b ein Bedienelement angezeigt, das nachfolgend als Kollaborationspilot 42 (FIG 4) bezeichnet ist.

In diesem Kollaborationspiloten 42, der beispielhaft als Fenster oder Rahmen innerhalb des jeweiligen GUI 20 dargestellt ist, werden die Identitäten derjenigen Benutzer angezeigt, die zu einem gemeinsamen Zeitpunkt durch Interaktion mit dem GUI 20 ihre Bereitschaft zur Teilnahme an einer Kollaborationssitzung erklärt haben. Jede Kollaborationssitzung ist hierbei durch eine Kopplung einer Präsentations-Pipeline 30 mit einer oder mehreren Betrachter-Pipelines 32 anderer Applikationen 15, und somit durch einen gemeinsamen auszuwertenden Bilddatensatz B charakterisiert. Bezüglich jeder Kollaborationssitzung kann sich einer der beteiligten Benutzer in dem Kollaborationspiloten 42 durch Interaktion mit dem GUI 20 als Präsentator ausweisen lassen. Der oder die weiteren Benutzer, der bzw. die die Bereitschaft zur Teilnahme an der Kollaborationssitzung erklärt haben, werden in dem Kollaborationspiloten 42 als Betrachter ausgewiesen.

Der beispielhaft als Präsentator ausgewiesene Benutzer der Applikation 15a kann durch Interaktion mit dem GUI 20 das dortige Segment 21 (oder ein beliebiges von ggf. mehreren in dem GUI 20 angezeigten Segmenten) als Präsentatorsegment 31 markieren, z.B. indem er ein seine Person repräsentierendes Icon in dem GUI 20 in das gewünschte Segment 21 zieht. Bei Erkennung diese Benutzerinteraktion schaltet die Applikation 15a das betreffende Segment 21 von dem Stand-Alone-Betrieb in den Kollaborationsbetrieb und aktiviert die zugehörige Pipeline 24 als Präsentator-Pipeline 30, indem sie gemäß FIG 5 den Koppelfilter 34 in die Pipeline 24 einhängt und die Einschubmodule 39 und 41 mit dem Befundverwaltungsmodul 27 bzw. dem Event-Handler 26 koppelt.

Ebenso kann der beispielhaft als Betrachter ausgewiesene Benutzer der Applikation 15b durch Interaktion mit dem GUI 20 das dortige Segment 21 (oder ein beliebiges von ggf. mehreren in dem GUI 20 angezeigten Segmenten) als Betrachtersegment 33 markieren, z.B. indem er ein seine Person repräsentierendes Icon in dem GUI 20 in das gewünschte Segment 21 zieht. Bei Erkennung dieser Benutzerinteraktion schaltet auch die Applikation 15b das betreffende Segment 21 von dem Stand-Alone-Betrieb in den Kollaborationsbetrieb und aktiviert die zugehörige Pipeline 24 als Betrachter-Pipeline 32, indem sie gemäß FIG 5 den Koppelfilter 35 in die Pipeline 24 einhängt und die Einschubmodule 38 und 40 mit dem Befundverwaltungsmodul 27 bzw. dem Event-Handler 26 koppelt.

Zwar kann das System 1 im Rahmen der Erfindung grundsätzlich derart gestaltet sein, dass die Applikationen 15a,15b direkt (Peer-to-Peer) Daten, insbesondere die teilbearbeiteten Daten T3 sowie betrachterseitig erzeugte Befunde F und Parameter P austauschen. In bevorzugter Ausbildung des Systems 1 kommuniziert aber, wie in FIG 4 dargestellt ist, jede der Applikationen 15a,15b in dem Kollaborationsbetrieb ausschließlich mit der zentralen Kollaborationseinheit 16, die den Datenaustausch zwischen den Applikationen 15a,15b vermittelt. Die zentrale Kollaborationseinheit 16 koordiniert hierbei auch die Kollaborationspiloten 42 der Applikationen 15a,15b.

In bevorzugter Ausbildung des Systems 1 ist jede der Applikationen 15 zur gleichzeitigen Ausführung mehrerer Kollaborationssitzungen ausgebildet. Mit anderen Worten sind die Applikationen 15 dazu eingerichtet, dass nach Wahl des Benutzers eines oder mehrere Segmente 21 des jeweiligen GUI 20 gleichzeitig - aber im Rahmen unterschiedlicher Kollaborationssitzungen - im Kollaborationsbetrieb betrieben werden können. In einer zweckmäßigen, aber optionalen Ausführung des Systems 1 sind die Applikationen 15 dabei derart ausgebildet, dass pro Applikation 15 zu demselben Zeitpunkt lediglich ein einziges Segment 21 als Präsentatorsegment 31 betreibbar ist.

Bevorzugt werden im Kollaborationsbetrieb zudem nicht nur die teilbearbeiteten Daten T3 des Bilddatensatzes B und Befunde F sowie Parameter P zwischen den beteiligten Applikationen 15a,15b ausgetauscht, sondern auch Tonsignale (aufgenommene Sprache) sowie optional Videosignale von ggf. vorhandenen Kameras der beteiligten Devices 2. Auch die Ton- bzw. Videoübertragung wird hierbei zweckmäßigerweise über die zentrale Kollaborationseinheit 16 zwischen den Applikationen 15a,15b vermittelt.

In FIG 6 sind die an der Realisierung des Kollaborationsbetrieb beteiligten Komponenten des Systems 1 noch einmal in einer Übersicht dargestellt.

Aus der Darstellung ist ersichtlich, dass jeder Applikation 15 eine lokale Kollaborationseinheit 50 zugeordnet ist, die die für die Kollaboration erforderlichen Funktionen und Komponenten vereint und mit der zentralen Kollaborationseinheit 16 kommuniziert. Die einer Applikation 15 zugeordnete lokale Kollaborationseinheit umfasst somit insbesondere die Koppelfilter 34,35, die Einschubmodule 38-41 sowie den Kollaborationspiloten 42). Wie vorstehend bereits angedeutet, sind die lokalen Kollaborationseinheit 50 vorzugsweise als separate Module ausgebildet, die - z.B. nach Art von Plug-ins - zu Laufzeit mit der jeweils zugeordneten Applikation 15 gekoppelt werden können. In alternativer - ebenfalls bereits erwähnter - Ausführung sind die lokalen Kollaborationseinheiten 50 als fester Bestandteil der jeweils zugeordneten Applikation 15a,15b implementiert.

Gemäß FIG 6 enthält jede lokale Kollaborationseinheit 50 einen Präsentatorkanal 51, über den einzelnen Segmenten 21 der zugehörigen Applikation 15 ihre jeweilige Rolle als Präsentatorsegment 31 bzw. Betrachtersegment 33 zugewiesen wird. Dieser Präsentatorkanal 51, der insbesondere auch den Kollaborationspiloten 42 steuert, kommuniziert mit einem als Präsentationsmanager 52 bezeichneten Modul der zentralen Kollaborationseinheit 16. Der Präsentationsmanger 52 koordiniert hierbei die Rollen der beteiligten Segmente 21 in den jeweils applikationsübergreifenden Kollaborationssitzungen.

Des Weiteren enthält jede lokale Kollaborationseinheit 50 einen Ereigniskanal 53, über den die für die Kollaboration relevanten Ereignisse zwischen den Applikationen 15 gemeldet werden. Diese Ereignisse umfassen insbesondere die Bereitstellung von teilbearbeiteten Daten T3 durch die Präsentator-Pipeline 30 sowie die betrachterseitige Erzeugung von neuen Befunden F und Parametern P. Dieser Ereigniskanal 53, der insbesondere mit Einschubmodulen 38-41 gemäß FIG 5 wechselwirkt, kommuniziert mit einem entsprechenden, als Ereignismanager 54 bezeichneten Modul der zentralen Kollaborationseinheit 16, wobei dieser Ereignismanager 54 die zugeführten Ereignisse applikationsübergreifend verwaltet und weiterleitet.

Der Austausch der mit diesen Ereignissen verbundenen Daten T3, Befunde F und Parameter P wird über ein als Datenaustauschmanager 55 bezeichnetes Modul der zentralen Kollaborationseinheit 16 vermittelt. Der Datenaustauschmanager 55 behandelt die auszutauschenden Daten unabhängig von der jeweiligen Datenart und ist in diesem Sinne formlos.

Schließlich enthält jede lokale Kollaborationseinheit 50 einen Audiokanal 56, über den im Zuge der Kollaboration aufgenommenen Tonsignale (und ggf. Videosignale) ausgetauscht werden. Dieser Audiokanal 56 kommuniziert mit einem entsprechenden, als Audiomanager 57 bezeichneten Modul der zentralen Kollaborationseinheit 16, wobei dieser Audiomanager 57 die zugeführten Tonsignale (und ggf. Videosignale) verwaltet und weiterleitet.

Die Erfindung wird an den vorstehend beschriebenen Ausführungsbeispielen besonders deutlich, ist gleichwohl auf diese Ausführungsbeispiele aber nicht beschränkt. Vielmehr können zahlreiche weitere Ausführungsformen der Erfindung aus den Ansprüchen und der vorstehenden Beschreibung abgeleitet werden. Insbesondere können im Rahmen der Ansprüche einzelne Merkmale der Ausführungsbeispiele weggelassen, anders kombiniert oder durch weitere Merkmale ergänzt werden, ohne von der Erfindung abzuweichen.

### Bezugszeichenliste

- 1: System
- 2: Device
- 3: (Datenübertragungs-)Netz
- 4: Rechner
- 5: Bildanzeigemittel
- 6: Eingabemittel
- 7: Befundungsstation
- 8: PC-Arbeitsplatz
- 9: Tabletcomputer
- 10: Smartphone
- 11: LAN
- 12: Drahtlosnetzwerk
- 13: Internet
- 14: Firewall
- 15,15a,15b: Applikation
- 16: (zentrale) Kollaborationseinheit
- 17: (Public) Cloud
- 18: Speicher
- 20: GUI
- 21: Segment
- 22: Frontend
- 23: Backend
- 24: (Bildbearbeitungs-)Pipeline
- 25: Filter
- 26: Event-Handler
- 27: Befundverwaltungsmodul
- 30: Präsentator-Pipeline
- 31: Präsentatorsegment
- 32: Betrachter-Pipeline
- 33: Betrachtersegment
- 34: Koppelfilter
- 35: Koppelfilter
- 36: Markierung
- 37: Markierung
- 38: Einschubmodul
- 39: Einschubmodul
- 40: Einschubmodul
- 41: Einschubmodul
- 42: Kollaborationspilot
- 50: (lokale) Kollaborationseinheit
- 51: Präsentationskanal
- 52: Präsentationsmanager
- 53: Ereigniskanal
- 54: Ereignismanager
- 55: Datenaustauschmanager
- 56: Audiokanal
- 57: Audiomanager

- B: Bilddatensatz
- V: Ansicht
- P: Parameter
- E: Ereignis
- I: Eingabe

- T1-T4: teilbearbeitete Daten
- T3`: teilbearbeitete Daten

## Patentansprüche

1. Verfahren zur gemeinsamen Auswertung eines medizinischen Bilddatensatzes (B) an einer ersten Datenverarbeitungseinrichtung (2) und mindestens einer damit über ein Datenübertragungsnetz (3) verbundenen zweiten Datenverarbeitungseinrichtung (2),
- wobei in der ersten Datenverarbeitungseinrichtung (2) eine erste Applikation (15a) und in der zweiten Datenverarbeitungseinrichtung (1) eine zweite Applikation (15b) ausgeführt werden,
- wobei jede dieser Applikationen (15a,15b) jeweils eine graphische Benutzeroberfläche (20) mit mindestens einem Segment (21) zur Anzeige einer Ansicht (V) des Bilddatensatzes (B) aufweist,
**dadurch gekennzeichnet,**
- **dass** diese Ansicht (V) mittels einer dem jeweiligen Segment (21) zugeordneten Bildbearbeitungs-Pipeline (24) in einem mehrstufigen Bearbeitungsprozess aus dem Bilddatensatz (B) abgeleitet wird,
- **dass** das Segment (21) der ersten Applikation (15a) als Präsentatorsegment (31) betrieben wird, indem, mittels eines Farbfilters der Bildbearbeitungs-Pipeline (24,30) des Präsentatorsegments (31) teilbearbeitete, Daten (T3) des Bilddatensatzes (B) vor Durchführung einer Formfaktoranpassung aus der Bildbearbeitungs-Pipeline (24,30) dieses Präsentatorsegments (31) ausgekoppelt und an die zweite Applikation (15b) übermittelt werden, und
- **dass** das Segment (21) der zweiten Applikation (15b) als Betrachtersegment (33) betrieben wird, indem die übermittelten, teilbearbeiteten Daten (T3) des Bilddatensatzes (B), zur Fertigstellung der Ansicht (V) mittels eines Filters der Bildbearbeitungs-Pipeline (24,32) des Betrachtersegments (33), vor einer Formfaktoranpassung in die Bildbearbeitungs-Pipeline (24,32) dieses Betrachtersegments (33) eingekoppelt werden.

2. Verfahren nach Anspruch 1,
wobei die Ansicht (V) durch Kombination von Bilddaten des Bilddatensatzes (B) mit Daten mindestens einer weiteren Datenart, umfassend Metadaten und/oder Befunde (F), erzeugt wird, und wobei die Bilddaten und die Daten der mindestens einen weiteren Datenart separat voneinander aus der Bildbearbeitungs-Pipeline (24,30) des Präsentatorsegments (31) ausgekoppelt und in die Bildbearbeitungs-Pipeline (24,32) des Betrachtersegments (33) eingekoppelt werden, und dort zu der Ansicht (V) kombiniert zu werden.

3. Verfahren nach einem der Ansprüche 1 bis 2,
wobei durch Benutzerinteraktion mit der Benutzeroberfläche (20) der zweiten Applikation (15b) in dem Betrachtersegment (33) ein Befund (F) erzeugt wird, und wobei dieser Befund (F) an die erste Applikation (15a) übertragen und dort in die Bildbearbeitungs-Pipeline (24,30) des Präsentatorsegments (31) eingespeist wird.

4. Verfahren nach einem der Ansprüche 1 bis 3,
wobei eines von mehreren Segmenten (21) der Benutzeroberfläche (20) der ersten Applikation (15a) durch Benutzerinteraktion mit dieser Benutzeroberfläche (20) als Präsentatorsegment (31) ausgewählt wird, und/oder wobei eines von mehreren Segmenten (21) der Benutzeroberfläche (20) der zweiten Applikation (15b) durch Benutzerinteraktion mit dieser Benutzeroberfläche (20) als Betrachtersegment (33) ausgewählt wird.

5. System (1) zur gemeinsamen Auswertung eines medizinischen Bilddatensatzes (B) mit mindestens einer ersten Datenverarbeitungseinrichtung (2) und mit mindestens einer zweiten Datenverarbeitungseinrichtung (2), sowie mit einem Datenübertragungsnetz (3), über welches die erste Datenverarbeitungseinrichtung (3) und die zweite Datenverarbeitungseinrichtung (2) verbunden sind,
- wobei in der ersten Datenverarbeitungseinrichtung (2) eine erste Applikation (15a) und in der zweiten Datenverarbeitungseinrichtung (2) eine zweite Applikation (15b) implementiert sind,
- wobei jede der beiden Applikationen (15a,15b) jeweils eine graphische Benutzeroberfläche (20) mit mindestens einem Segment (21) zur Anzeige einer Ansicht (V) des Bilddatensatzes (B) aufweist,
**dadurch gekennzeichnet,**
- **dass** jedem Segment (21) eine Bildbearbeitungs-Pipeline (24) zur Erzeugung der Ansicht (V) aus dem Bilddatensatz (B) in einem mehrstufigen Bearbeitungsprozess zugeordnet ist,
- **dass** die erste Applikation (15a) dazu eingerichtet ist, mittels eines Farbfilters der Bildbearbeitungs-Pipeline (24,30) des Präsentatorsegments (31) teilbearbeitete, Daten (T3) des Bilddatensatzes (B) vor einer Formfaktoranpassung aus der Bildbearbeitungs-Pipeline (24,30) des zugehörigen Segments (21) auszukoppeln und an die zweite Applikation (15b) zu übermitteln, um dieses Segment (21) somit als Präsentatorsegment (31) zu betreiben, und
- **dass** die zweite Applikation (15b) dazu eingerichtet ist, die übermittelten, teilbearbeiteten Daten (T3) des Bilddatensatzes (B) zu empfangen und, zur Fertigstellung der Ansicht (V) mittels eines Filters der Bildbearbeitungs-Pipeline (24,32) des Betrachtersegments (33), vor einer Formfaktoranpassung in die Bildbearbeitungs-Pipeline (24,32) des zugehörigen Segments (21) einzukoppeln, um dieses Segment (21) somit als Betrachtersegment (33) zu betreiben.

6. System (1) nach Anspruch 5,
- wobei die Bildbearbeitungs-Pipeline (24,30) des Präsentatorsegments (31) und die Bildbearbeitungs-Pipeline (24,32) des Betrachtersegments (33) jeweils dazu eingerichtet sind, die Ansicht (V) durch Kombination von Bilddaten des Bilddatensatzes (B) mit Daten mindestens einer weiteren Datenart, umfassend Metadaten und/oder Befunde (F), zu erzeugen,
- wobei die erste Applikation (15a) dazu eingerichtet ist, die Bilddaten und die Daten der mindestens einen weiteren Datenart separat voneinander aus der Bildbearbeitungs-Pipeline (24,30) des Präsentatorsegments (31) auszukoppeln, und
- wobei die zweite Applikation (15b) dazu eingerichtet ist, die Bilddaten und die Daten der mindestens einen weiteren Datenart separat voneinander in die Bildbearbeitungs-Pipeline (32) des Betrachtersegments (33) einzukoppeln und dort zu der Ansicht (V) zu kombinieren.

7. System (1) nach einem der Ansprüche 5 bis 6,
- wobei die zweite Applikation (15b) dazu eingerichtet ist, dass durch Benutzerinteraktion mit der zugehörigen Benutzeroberfläche (20) in dem Betrachtersegment (33) ein Befund (F) erzeugbar ist, und wobei die zweite Applikation (15b) dazu eingerichtet ist, diesen Befund (F) an die erste Applikation (15a) zu übertragen, und
- wobei die erste Applikation (15a) dazu eingerichtet ist, diesen Befund (15a) in die Bildbearbeitungs-Pipeline (24,30) des Präsentatorsegments (31) einzuspeisen.

8. System (1) nach einem der Ansprüche 5 bis 7,
- wobei die Benutzeroberfläche (20) der ersten Applikation (15a) mehrere Segmente (21) umfasst, wobei jedes dieser Segmente (21) durch Benutzerinteraktion mit dieser Benutzeroberfläche (20) als Präsentatorsegment (31) auswählbar ist, und/oder
- wobei die Benutzeroberfläche (20) der zweiten Applikation (15b) mehrere Segmente (21) umfasst, wobei jedes dieser Segmente (21) durch Benutzerinteraktion mit dieser Benutzeroberfläche (20) als Betrachtersegment (33) auswählbar ist.

9. System (1) nach einem der Ansprüche 5 bis 8,
mit einer zentralen Kollaborationseinheit (16), die die Datenübermittlung zwischen der ersten Applikation (15a) und der mindestens einen zweiten Applikation (15a) vermittelt.

10. System (1) nach Anspruch 9,
wobei die zentrale Kollaborationseinheit (10) in einer Cloud (17) implementiert ist.

## Claims

1. Method for joint evaluation of a medical image dataset (B) on a first data processing device (2) and at least one second data processing device (2) which is connected thereto by way of a data transmission network (3),
- wherein a first application (15a) is performed in the first data processing device (2) and a second application (15b) is performed in the second data processing device (1),
- wherein each of these applications (15a, 15b) has a respective graphical user interface (20) having at least one segment (21) for display of a view (V) of the image dataset (B),
**characterised in that**
- this view (V) is derived from the image dataset (B) by means of an image processing pipeline (24) that is associated with the respective segment (21) in a multi-stage processing process,
- the segment (21) of the first application (15a) is operated as a presenter segment (31) **in that** data (T3), which is partially processed by means of a colour filter of the image processing pipeline (24, 30) of the presenter segment (31), of the image dataset (B) is decoupled from the image processing pipeline (24, 30) of this presenter segment (31) before form factor adjustment has been performed and is transferred to the second application (15b), and
- the segment (21) of the second application (15b) is operated as an observer segment (33) **in that** the transferred partially processed data (T3) of the image dataset (B) is coupled to the image processing pipeline (24, 32) of this observer segment (33) before form factor adjustment for preparing the view (V) by means of a filter of the image processing pipeline (24, 32) of the observer segment (33).

2. Method according to claim 1,
wherein the view (V) is generated by combining image data of the image dataset (B) with data of at least one further data type, including metadata and/or assessments (F), and wherein the image data and the data of the at least one further data type are decoupled separately from one another from the image processing pipeline (24, 30) of the presenter segment (31) and coupled to the image processing pipeline (24, 32) of the observer segment (33) and are combined there to give the view (V) .

3. Method according to one of claims 1 to 2,
wherein, by user interaction with the user interface (20) of the second application (15b), an assessment (F) is generated in the observer segment (33), and wherein this assessment (F) is transferred to the first application (15a) and there fed into the image processing pipeline (24, 30) of the presenter segment (31).

4. Method according to one of claims 1 to 3,
wherein one of a plurality of segments (21) of the user interface (20) of the first application (15a) is selected as the presenter segment (31) by user interaction with this user interface (20), and/or
wherein one of a plurality of segments (21) of the user interface (20) of the second application (15b) is selected as the observer segment (33) by user interaction with this user interface (20).

5. System (1) for joint evaluation of a medical image dataset (B) having at least a first data processing device (2) and at least a second data processing device (2), and having a data transmission network (3) by way of which the first data processing device (3) and the second data processing device (2) are connected,
- wherein a first application (15a) is implemented in the first data processing device (2) and a second application (15b) is implemented in the second data processing device (2),
- wherein each of the two applications (15a, 15b) has a respective graphical user interface (20) having at least one segment (21) for display of a view (V) of the image dataset (B),
**characterised in that**
- an image processing pipeline (24) for generating the view (V) from the image dataset (B) is associated with each segment (21) in a multi-stage processing process,
- the first application (15a) is set up to decouple data (T3), which is partially processed by means of a colour filter of the image processing pipeline (24, 30) of the presenter segment (31), of the image dataset (B) from the image processing pipeline (24, 30) of the associated segment (21) before form factor adjustment and to transfer it to the second application (15b) in order thus to operate this segment (21) as a presenter segment (31), and
- the second application (15b) is set up to receive the transferred partially processed data (T3) of the image dataset (B) and to couple it to the image processing pipeline (24, 32) of the associated segment (21) before form factor adjustment, for preparing the view (V) by means of a filter of the image processing pipeline (24, 32) of the observer segment (33), in order thus to operate this segment (21) as an observer segment (33).

6. System (1) according to claim 5,
- wherein the image processing pipeline (24, 30) of the presenter segment (31) and the image processing pipeline (24, 32) of the observer segment (33) are each set up to generate the view (V) by combining image data of the image dataset (B) with data of at least one further data type, including metadata and/or assessments (F),
- wherein the first application (15a) is set up to decouple the image data and the data of the at least one further data type separately from one another from the image processing pipeline (24, 30) of the presenter segment (31), and
- wherein the second application (15b) is set up to couple the image data and the data of the at least one further data type separately from one another to the image processing pipeline (32) of the observer segment (33) and to combine them there to give the view (V).

7. System (1) according to one of claims 5 to 6,
- wherein the second application (15b) is set up such that, by user interaction with the associated user interface (20), an assessment (F) may be generated in the observer segment (33), and wherein the second application (15b) is set up to transfer this assessment (F) to the first application (15a), and
- wherein the first application (15a) is set up to feed this assessment (15a) into the image processing pipeline (24, 30) of the presenter segment (31).

8. System (1) according to one of claims 5 to 7,
- wherein the user interface (20) of the first application (15a) includes a plurality of segments (21), wherein each of these segments (21) is selectable as the presenter segment (31) by user interaction with this user interface (20), and/or
- wherein the user interface (20) of the second application (15b) includes a plurality of segments (21), wherein each of these segments (21) may be selected as the observer segment (33) by user interaction with this user interface (20).

9. System (1) according to one of claims 5 to 8,
having a central collaboration unit (16) that brings about the data transfer between the first application (15a) and the at least one second application (15a).

10. System (1) according to claim 9,
wherein the central collaboration unit (10) is implemented in a cloud (17).

## Revendications

1. Procédé d'évaluation conjointe d'un ensemble (B) de données d'image médicale à un premier dispositif (2) de traitement de données et à au moins un deuxième dispositif (2) de traitement de données, qui y est relié par un réseau (3) de transmission de données,
- dans lequel on exécute, dans le premier dispositif (2) de traitement de données, une première application (15a) et, dans le deuxième dispositif (1) de traitement de données, une deuxième application (15b),
- dans lequel chacune de ces applications (15a, 15b) a respectivement une surface (20) graphique d'utilisateur ayant au moins un segment (21) pour l'affichage d'une vue (V) de l'ensemble (B) de données d'image,
**caractérisé**
- **en ce que** l'on déduit cette vue (V) à partir de l'ensemble (B) de données d'image dans un processus de traitement en plusieurs stades, au moyen d'un pipeline (24) de traitement d'image affecté au segment (21) respectif,
- **en ce que** l'on fait fonctionner le segment (21) de la première application (15a) comme segment (31) de présentateur par le fait que, au moyen d'un filtre coloré du pipeline (24, 30) de traitement d'image du segment (31) de présentateur, on extrait du pipeline (24, 30) de traitement d'image de ce segment (31) de présentateur, des données (T3) traitées partiellement de l'ensemble (B) de données d'image avant d'effectuer une adaptation de facteur de forme et on les transmet à la deuxième application (15b), et
- **en ce que** l'on fait fonctionner le segment (21) de la deuxième application (15b) comme segment (33) observateur par le fait que l'on insère dans le pipeline (24, 32) de traitement d'image de ce segment (33) observateur, avant une adaptation de facteur de forme, les données (T3) transmises et traitées partiellement de l'ensemble (B) de données d'image pour la mise à disposition de la vue (V), au moyen d'un filtre du pipeline (24, 32) de traitement d'image du segment (33) observateur.

2. Procédé suivant la revendication 1,
dans lequel on produit la vue (V) par combinaison de données d'image de l'ensemble (B) de données d'image avec des données d'au moins un autre type de données, comprenant des métadonnées et/ou des constatations (F), et dans lequel on extrait du pipeline (24, 30) de traitement d'image du segment (31) présentateur, séparément les unes des autres, les données d'image et les données du au moins un autre type de données, et on les insère dans le pipeline (24, 32) de traitement d'image du segment (33) observateur et on les y combine en la vue (V).

3. Procédé suivant l'une des revendications 1 à 2,
dans lequel par interaction de l'utilisateur avec la surface (20) d'utilisateur de la deuxième application (15b), on produit dans le segment (33) observateur une constatation (F) et dans lequel on transmet cette constatation (F) à la première application (15a) et on l'y injecte dans le pipeline (24, 30) de traitement d'image du segment (31) présentateur.

4. Procédé suivant l'une des revendications 1 à 3,
dans lequel on choisit comme segment (31) présentateur l'un de plusieurs segments (21) de la surface (20) d'utilisateur de la première application (15a) par interaction de l'utilisateur avec cette surface (20) d'utilisateur et/ou
dans lequel on choisit comme segment (33) observateur l'un des plusieurs segments (21) de la surface (20) d'utilisateur de la deuxième application (15b) par interaction de l'utilisateur avec cette surface (20) d'utilisateur.

5. Système (1) d'évaluation conjointe d'un ensemble (B) de données d'images médicales, comprenant au moins un premier dispositif (2) de traitement de données et comprenant au moins un deuxième dispositif (2) de traitement de données ainsi qu'un réseau (3) de transmission de données, par lequel le premier dispositif (2) de traitement de données et le deuxième dispositif (2) de traitement de données communiquent,
- dans lequel une première application (15a) est mise en oeuvre dans le premier dispositif (2) de traitement de données et une deuxième application (15b) est mise en oeuvre dans le deuxième dispositif (2) de traitement de données,
- dans lequel chacune des deux applications (15a, 15b) a respectivement une surface (20) graphique d'utilisateur ayant au moins un segment (21) pour l'affichage d'une vue (V) de l'ensemble (B) de données d'image,
**caractérisé**
- **en ce qu'**à chaque segment (21) est associé un pipeline (24) de traitement d'image pour la production de la vue (V) à partir de l'ensemble (B) de données d'image dans un processus de traitement à plusieurs stades,
- **en ce que** la première application (15a) est agencée pour, au moyen d'un filtre coloré du pipeline (24, 30) de traitement d'image du segment (31) présentateur, extraire du pipeline (24, 30) de traitement d'image du segment (21) associé, avant une adaptation de facteur de forme, des données (T3) traitées partiellement de l'ensemble (B) de données d'image et pour les transmettre à la deuxième application (15b) afin de faire fonctionner ainsi ce segment (21) comme segment (31) présentateur, et
- **en ce que** la deuxième application (15b) est agencée pour recevoir des données (T3) transmises traitées partiellement de l'ensemble (B) de données d'image et pour, pour la mise à disposition de la vue (V) au moyen d'un filtre du pipeline (24, 32) de traitement d'image du segment (33) observateur, les insérer dans le pipeline (24, 32) de traitement d'image du segment (21) associé avant une adaptation de facteur de forme, afin de faire ainsi fonctionner ce segment (21) comme segment (33) observateur.

6. Système (1) suivant la revendication 5,
- dans lequel le pipeline (24, 30) de traitement d'image du segment (31) présentateur et le pipeline (24, 32) de traitement d'image du segment (33) observateur sont agencés respectivement pour produire la vue (V) par combinaison de données d'image de l'ensemble (B) de données d'image avec des données du au moins un autre type de données, comprenant des métadonnées et/ou des constatations (F),
- dans lequel la première application (15a) est agencée pour extraire les données d'image et les données du au moins un autre type de données, séparément les unes des autres, du pipeline (24, 30) de traitement d'image du segment (31) présentateur, et
- dans lequel la deuxième application (15b) est agencée pour introduire dans le pipeline (32) de traitement d'image du segment (33) observateur, séparément les unes des autres, des données d'image et des données du au moins un autre type de données et pour les y combiner en la vue (V).

7. Système (1) suivant l'une des revendications 5 à 6,
- dans lequel la deuxième application (15b) est agencée, de manière à pouvoir produire par interaction de l'utilisateur avec la surface (20) d'utilisateur associée dans le segment (33) d'observateur, une constatation (F) et dans lequel la deuxième application (15b) est agencée pour transmettre cette constatation (F) à la première application (15a), et
- dans lequel la première application (15a) est agencée pour introduire cette constatation (15a) dans le pipeline (24, 30) de traitement d'image du segment (31) présentateur.

8. Système (1) suivant l'une des revendications 5 à 7,
- dans lequel la surface (20) d'utilisateur de la première application (15a) comprend plusieurs segments (21), dans lequel chacun de ces segments (21) peut être sélectionné comme segment (31) présentateur par interaction de l'utilisateur avec cette surface (20) d'utilisateur, et/ou
- dans lequel la surface (20) d'utilisateur de la deuxième application (15b) comprend plusieurs segments (21), dans lequel chacun de ces segments (21) peut être sélectionné comme segment (33) observateur par interaction de l'utilisateur avec cette surface (20) d'utilisateur.

9. Système (1) suivant l'une des revendications 5 à 8,
comprenant une unité (16) centrale de collaboration, qui assure la transmission de données entre la première application (15a) et la au moins une autre application (15a) .

10. Système (1) suivant la revendication 9,
dans lequel l'unité (10) centrale de collaboration est mise en œuvre dans un nuage (17).
